# DEMANDE DE BREVET EUROPEEN

(11) **EP 4 186 476 A1**
(43) Date de publication de la demande: **31.05.2023**
(21) Numéro de dépôt: 22209500.2
(22) Date de dépôt: 24.11.2022
(51) Int. Cl.: A61F 2/30

(54) **PROCÉDÉ POUR LA FABRICATION D'UN IMPLANT DE PROTHÈSE ARTICULAIRE À L AIDE D'UNE EMPREINTE EN CÉRAMIQUE**

(30) Priorité: 26.11.2021 FR 2112612
(71) Demandeur: FX Solutions, 01000 Bourg en Bresse (FR)
(72) Inventeur: DAUDET, Jean-Marie, 01000 BOURG-EN-BRESSE (FR)
(74) Mandataire: LLR

(57) **Abrégé**

L'invention concerne un procédé et un dispositif de fabrication d'un implant de prothèse articulaire à l'aide d'un moule comprenant une empreinte en céramique (1) mise en mouvement par un piston (2), l'empreinte en céramique (1) venant presser un matériau thermoplastique introduit au sein du moule.

## Description

L'invention concerne un procédé pour la fabrication d'un implant de prothèse articulaire et un dispositif permettant la mise en oeuvre dudit procédé.

On connaît déjà dans l'état la technique des procédés de fabrication d'implant de prothèse articulaire dont au moins une partie est obtenue par moulage ou surmoulage d'un matériau thermoplastique. Pour ce faire, le matériau thermoplastique est introduit dans l'empreinte du moule, cette dernière étant généralement métallique (en inox par exemple), en vue d'être moulée ou surmoulée sur un élément de support préalablement introduit au sein du moule de l'implant. Le fluage de la matière thermoplastique en vue d'obtenir sa forme définitive est généralement réalisé par thermocompression ou injection de la matière thermoplastique au sein de l'empreinte du moule. Un inconvénient de ce procédé de fabrication est que l'étape d'extraction de l'implant obtenu est très compliquée à mettre en œuvre compte tenu de la très forte adhérence entre le matériau thermoplastique et l'empreinte métallique. En conséquence, il existe un risque que l'implant extrait du moule ne respecte plus le dimensionnement souhaité avant fabrication. Une mauvaise extraction de l'implant ainsi obtenu risque également d'entrainer des propriétés mécaniques plus faibles que celles souhaitées avant fabrication.

Dès lors, une solution connue pour remédier à cet inconvénient consiste à appliquer (préalablement à l'étape de moulage ou de surmoulage du matériau thermoplastique) un agent démoulant l'empreinte du moule, permettant ainsi une extraction plus aisée et sécurisée de l'implant obtenu. Un tel agent démoulant est un matériau chimique connu de l'homme de métier. Toutefois, cette solution rallonge le procédé de fabrication de l'implant puisqu'une étape supplémentaire d'application de l'agent démoulant sur l'empreinte est requise ce qui augmente, in fine, le coût de fabrication de l'implant réalisé (l'agent démoulant étant un composant onéreux). Il convient d'envisager des procédés permettant une aussi bonne qualité d'extraction de l'implant sans devoir recourir à l'utilisation d'un tel agent démoulant, a fortiori au regard de la pénurie actuelle de matières premières.

L'invention a notamment pour but de proposer un procédé de fabrication d'un implant de prothèse articulaire ainsi qu'un dispositif permettant la mise en œuvre de ce procédé, qui surmontent l'ensemble des inconvénients précités.

A cet effet l'invention a pour objet un procédé de fabrication comprenant les étapes suivantes :
- porter un matériau thermoplastique destiné à constituer au moins partiellement l'implant à une température de malléabilité ;
- à l'intérieur d'une cavité d'un moule et à l'aide d'une empreinte en céramique du moule mise en mouvement par un piston, presser le matériau thermoplastique de façon à faire fluer ce matériau au sein de la cavité du moule afin qu'il épouse la forme de l'empreinte ;
- laisser refroidir le matériau thermoplastique ;
- extraire l'implant obtenu hors du moule.

Un tel procédé de fabrication permet d'assurer une production rapide et sécurisée, le tout en réduisant les coûts de fabrication puisqu'il n'est plus nécessaire de recourir à un agent démoulant. Il permet également de garantit la bonne qualité des implants obtenus.

Suivant d'autres caractéristiques optionnelles du procédé prises seules ou en combinaison :
- le matériau thermoplastique est choisi parmi : du polyéthylène de masse molaire très élevée (UHMWPE), du polyétheréthercétone (PEEK), du PEKK(poly ether ketone ketone) et leurs dérivés ;
- on introduit au sein de la cavité du moule une embase métallique contre laquelle est pressé le matériau thermoplastique ;
- la céramique est choisie parmi : alumine, composite, zircone ou oxynium.

L'invention a également pour objet un dispositif de fabrication d'un implant de prothèse articulaire comprenant :
- au moins un moule comprenant une empreinte en céramique ;
- au moins un piston configuré pour mettre en mouvement l'empreinte en céramique entre au moins deux positions prédéfinies, de sorte que dans l'une de ces positions l'empreinte en céramique presse un matériau thermoplastique préalablement introduit dans la cavité du moule et que dans l'autre position l'empreinte en céramique n'est pas au contact dudit matériau thermoplastique.

Suivant d'autres caractéristiques optionnelles du dispositif prises seules ou en combinaison :
- le piston est en céramique ;
- le piston et l'empreinte forme un élément monobloc ;
- la céramique est choisie parmi : alumine, composite, zircone ou oxynium ;
- le dispositif comprend cinq moules comprenant chacun une empreinte en céramique et cinq pistons configurés pour mettre en mouvement chacun une desdites empreintes.

### Brève description des figures

L'invention sera mieux comprise à la lecture de la description qui va suivre donnée uniquement à titre d'exemple et faite en se référant aux dessins annexés dans lesquels :
[Fig. 1] la figure 1 est une vue de côté d'une empreinte selon un premier mode de réalisation du dispositif de l'invention ;
[Fig. 2] la figure 2 est une vue en coupe de l'empreinte de la figure 1 ;
[Fig. 3] la figure 3 est une vue de côté de l'empreinte et du piston selon le premier mode de réalisation du dispositif de l'invention ;
[Fig. 4] la figure 4 est une vue en coupe de l'empreinte et du piston de la figure 3 ;
[Fig. 5] la figure 5 est une vue en perspective de l'empreinte et du piston de la figure 3 ;
[Fig. 6] la figure 6 est une vue de côté d'une empreinte et d'un piston selon un deuxième mode de réalisation du dispositif de l'invention ;
[Fig. 7] la figure 7 est une vue en coupe de l'empreinte et du piston de la figure 6 ;
[Fig. 8] la figure 8 est une vue en perspective d'un implant de prothèse articulaire obtenu
[Fig. 9] la figure 9 est une vue de côté d'une embase métallique de l'implant de la figure 8.

### Description détaillée

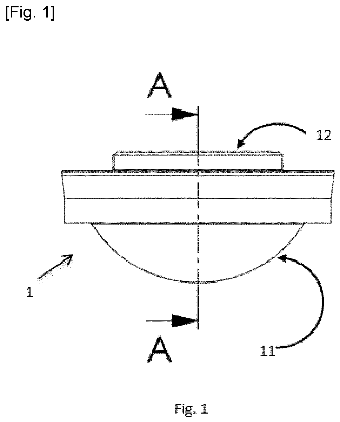

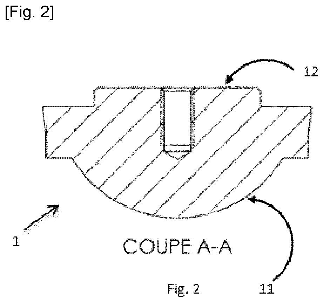

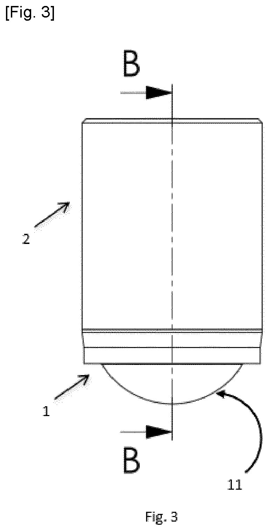

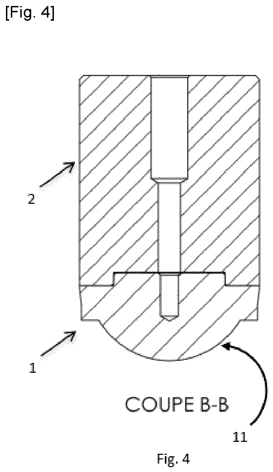

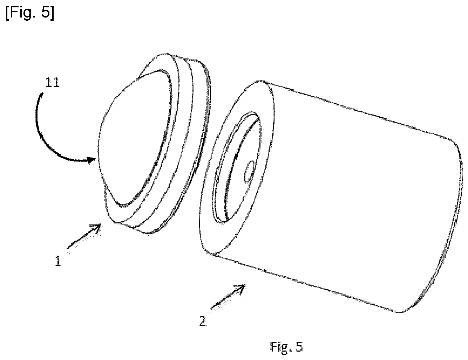

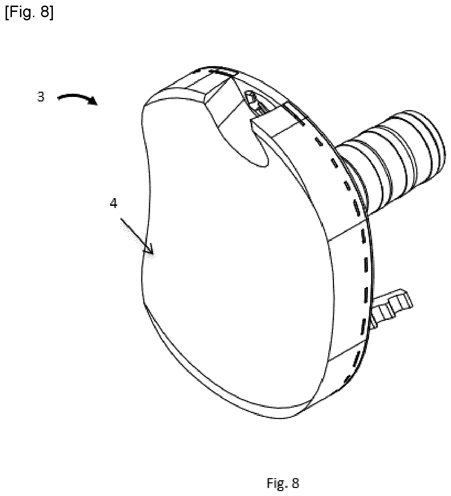

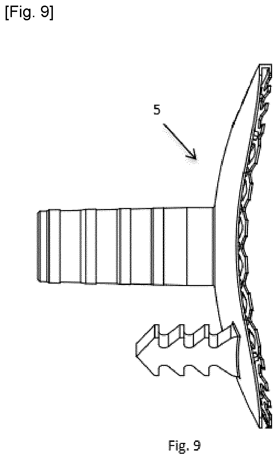

On a représenté sur les figures 1 à 7, une empreinte en céramique 1 et un piston 2 faisant parties du dispositif de fabrication d'un implant de prothèse articulaire selon l'invention, un exemple d'implant obtenue 3 étant visible sur les figures 8 et 9.

Le dispositif de fabrication d'un implant de prothèse articulaire selon l'invention comprend
- au moins un moule comprenant une empreinte en céramique 1 ;
- au moins un piston 2 configuré pour mettre en mouvement l'empreinte en céramique 1 entre au moins deux positions prédéfinies, de sorte que dans l'une de ces positions l'empreinte en céramique 1 presse un matériau thermoplastique préalablement introduit dans la cavité du moule et que dans l'autre position l'empreinte en céramique 1 n'est pas au contact dudit matériau thermoplastique.

L'empreinte en céramique 1 présente une surface 11 destinée à venir en contact avec la matière thermoplastique pressée de sorte à former l'implant articulaire 3. Elle présente en outre une butée 12 contre laquelle prend appuie le piston 2 lors de la mise en mouvement de l'empreinte 1.

Le piston 2 permet de commander directement la mise en mouvement de l'empreinte en céramique 1 au sein de la cavité du moule de sorte à presser la matière thermoplastique afin de former au moins partiellement l'implant 3.

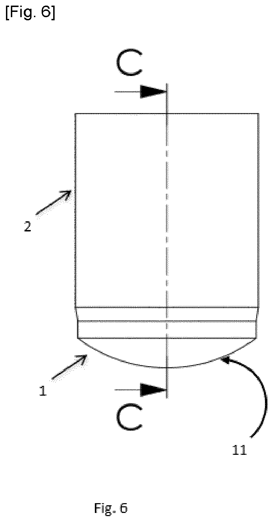

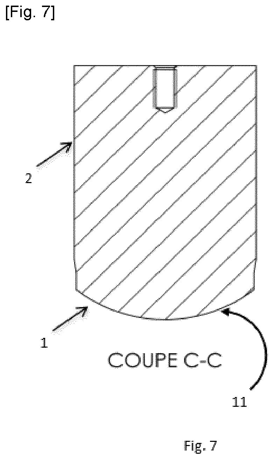

Selon un deuxième mode de réalisation du dispositif de fabrication (figures 6 et 7), l'empreinte 1 et le piston 2 sont réalisés en céramique et sont monobloc.

Un exemple d'implant 3 ainsi formé est représenté à la figure 8. Selon cet exemple, le matériau thermoplastique pressé forme le plateau de glissement 4 de l'implant 3, ce plateau étant surmoulé sur une embase métallique 5 (figure 9) préalablement placée dans le moule du dispositif de fabrication. Alternativement, il est tout à fait envisageable d'obtenir un implant entière réalisé à partir du matériau thermoplastique pressé au sein du moule.

Le procédé de fabrication d'un implant 3 de prothèse articulaire selon l'invention fonctionne comme suit :
- on porte le matériau thermoplastique destiné à constituer au moins partiellement l'implant 3 à une température de malléabilité ;
- à l'intérieur d'une cavité d'un moule et à l'aide d'une empreinte en céramique 1 du moule mise en mouvement par un piston 2, on presse le matériau thermoplastique de façon à faire fluer ce matériau au sein de la cavité du moule afin qu'il épouse la forme de l'empreinte 1 ;
- on laisser refroidir le matériau thermoplastique ;
- on extrait l'implant obtenu hors du moule.

Avantageusement, lorsqu'une embase métallique 5 est nécessaire pour former l'implant 3, on introduit au sein de la cavité du moule l'embase métallique 5 et on presse le matériau thermoplastique contre cette dernière.

Le matériau thermoplastique peut être tout type de matériau thermoplastique connu de l'homme du métier, par exemple : du polyéthylène de masse molaire très élevée (UHMWPE), du polyétheréthercétone (PEEK), du PEKK(poly ether ketone ketone) et leurs dérivés.

La céramique utilisée pour former l'empreinte 1 et le piston 2 peut être tout type de céramique connue de l'homme du métier, par exemple : alumine, composite, zircone ou oxynium.

L'invention n'est pas limitée aux modes de réalisation présentés et d'autres modes de réalisation apparaîtront clairement à l'homme du métier.

## Revendications

1. Procédé de fabrication d'un implant (3) de prothèse articulaire comprenant les étapes suivantes :
- porter un matériau thermoplastique destiné à constituer au moins partiellement l'implant (3) à une température de malléabilité ;
- à l'intérieur d'une cavité d'un moule et à l'aide d'une empreinte en céramique (1) du moule mise en mouvement par un piston (2), presser le matériau thermoplastique de façon à faire fluer ce matériau au sein de la cavité du moule afin qu'il épouse la forme de l'empreinte (1);
- laisser refroidir le matériau thermoplastique ;
- extraire l'implant (3) obtenu hors du moule.

2. Procédé de fabrication selon la revendication précédente, dans lequel le matériau thermoplastique est choisi parmi : du polyéthylène de masse molaire très élevée (UHMWPE), du polyétheréthercétone (PEEK), du PEKK(poly ether ketone ketone) et leurs dérivés.

3. Procédé de fabrication selon l'une quelconque des revendications précédentes, dans lequel on introduit au sein de la cavité du moule une embase métallique (5) contre laquelle est pressé le matériau thermoplastique.

4. Procédé de fabrication selon l'une quelconque des revendications précédentes, dans lequel la céramique est choisie parmi : alumine, composite, zircone ou oxynium.

5. Dispositif de fabrication d'un implant (3) de prothèse articulaire comprenant :
- au moins un moule comprenant une empreinte en céramique (1);
- au moins un piston (2) configuré pour mettre en mouvement l'empreinte en céramique (1) entre au moins deux positions prédéfinies, de sorte que dans l'une de ces positions l'empreinte en céramique (1) presse un matériau thermoplastique préalablement introduit dans la cavité du moule et que dans l'autre position l'empreinte en céramique (1) n'est pas au contact dudit matériau thermoplastique.

6. Dispositif de fabrication selon la revendication précédente, dans lequel le piston (2) est en céramique.

7. Dispositif de fabrication selon la revendication précédente, dans lequel le piston (2) et l'empreinte (1) forme un élément monobloc.

8. Dispositif de fabrication selon l'une des revendications 5 à 7, dans lequel la céramique est choisie parmi : alumine, composite, zircone ou oxynium.

9. Dispositif de fabrication selon l'une des revendications 5 à 7 comprenant cinq moules comprenant chacun une empreinte en céramique (1) et cinq pistons (2) configurés pour mettre en mouvement chacun une desdites empreintes (1).
